# EUROPEAN PATENT APPLICATION

(11) **EP 2 308 565 A2**
(43) Date of publication of application: **13.04.2011**
(21) Application number: 11151708.2
(22) Date of filing: 21.12.2005
(51) Int. Cl.: A61Q 11/02, A61Q 11/00, A61K 8/19, A61K 8/21, A61K 8/25, A61K 8/27, A61K 8/29, A61K 8/49, A61K 8/81, A61P 29/00

(54) **ORAL CARE COMPOSITIONS CONTAINING FREE-B-RING FLAVONOIDS AND FLAVANS**

(30) Priority: 22.12.2004 US 639331 P; 12.12.2005 US 301098
(62) Divisional of application: 05855133.4
(71) Applicant: Colgate-Palmolive Company, New York NY 10022-7499 (US)
(72) Inventor: Xu, Guofeng, Princeton, NJ 08542 (US); Boyd, Thomas, J., Metuchen, NJ 08840 (US); Hao, Zhigang, North Brunswick, NJ 08902 (US); Viscio, David, Monmouth Junction, NJ 08852 (US); Gaffar, Abdul, Princeton, NJ 08540 (US); Mello, Sarita V., Somerset, NJ 08873 (US); Arvanitidou, Evangelia S., Princeton, NJ 08540 (US); Prencipe, Michael, West Windsor, NJ 08550 (US)
(74) Representative: Jenkins, Peter David

(57) **Abstract**

Oral care compositions containing: a free-B-ring flavonoid and a flavan; as well as at least one bioavailability-enhancing agent are provided. Methods of using the oral compositions are also provided.

## Description

This application claims priority to United States Provisional Patent Application Serial No. 60/639,331, filed 22 December 2004, the contents of which are incorporated herein by reference in their entirety.

### BACKGROUND OF THE INVENTION

Oral inflammation is associated with common oral conditions, including periodontitis, for example. Gingivitis is the initial stage of gum disease. A cause of gingivitis is plaque, which is a soft, sticky, colorless film of bacteria that forms on the teeth and gums. Plaque, if left untreated, produces toxins that can inflame or infect the gum tissue to cause gingivitis. Untreated gingivitis can eventually spread from the gums to the ligaments and bone that support the teeth, and can cause periodontitis. While a variety of different treatments exist for preventing and suppressing oral inflammatory conditions, such treatments are subject to improvement.

### BRIEF SUMMARY OF THE INVENTION

In various embodiments, the invention provides an oral composition comprising a free-B-ring flavonoid and a flavan. Further, the composition comprises a bioavailability-enhancing agent for the flavonoid and/or the flavan.

In various embodiments, the oral composition comprises a free-B-ring flavonoid, a flavan, and optionally, an oral care active agent.

In certain embodiments, an oral composition comprises a free-B-ring flavonoid, a flavan, a bioavailability-enhancing agent for the flavonoid and/or the flavan; and a non-ionic antibacterial agent.

In other embodiments, an oral composition comprises a free-B-ring flavonoid, a flavan, and an oral care active agent comprising a cationic compound.

In yet other embodiments, an oral composition comprises a free-B-ring flavonoid, a flavan, and an oral care active agent comprising a stannous ion source.

Also provided are methods for inhibiting and/or treating an oral inflammatory condition in a mammalian subject. In various embodiments, the method comprises administering to the oral cavity of the subject an effective amount of an oral composition comprising a free-B-ring flavonoid, a flavan, and a bioavailability-enhancing agent for increasing the bioavailability of the flavonoid and/or the flavan in the oral tissue in the oral cavity.

### DETAILED DESCRIPTION OF THE INVENTION

It has been discovered that compositions and methods of this invention afford benefits including one or more of enhanced efficacy in treatment of oral inflammatory conditions, enhanced efficacy in inhibiting oral inflammatory conditions, and reduced side effects.

Oral compositions according to the present invention include one or more free-B-ring flavonoids and one or more flavans. In various embodiments, such compositions for topical (or injectable) oral administration provide a reduction of oral inflammation. The term oral surface and oral tissue encompass hard and soft tissues within the oral cavity. Hard tissues include the teeth, periodontal support, and the like. Soft tissues comprise gums, the tongue, surfaces of the buccal cavity and the like.

The present invention provides oral care compositions and methods for administration or application to, or use with, mammalian subjects, such as a human or animal.

The present invention, in various embodiments, provides oral compositions for treating and/or inhibiting various oral inflammatory conditions, such as gingivitis, periodontitis, oral lichen planus, Sjögren's syndrome, etc. The oral compositions can be present in various different forms. For example, the oral compositions can be at least one of a dentifrice, paste, gel, powder, mouth rinse, mouthwash, tooth hardener, oral film, anticalculus composition, film, slurry, injectable solution, and lozenge.

In various embodiments, oral compositions comprise at least one free-B-ring flavonoid and at least one flavan. The oral composition preferably also includes a bio-availability enhancing agent. Optionally at least one oral care active agent may be included. "Bioavailability-enhancing agent" refers to one or more constituents that are present in the oral composition that improve the degree to which an oral care active compound or other substance becomes available to the target tissue after administration to the oral cavity. In various embodiments, the bioavailability-enhancing agent improves the availability of the flavan and the flavonoid to the target oral tissue. Preferably, the bioavailability-enhancing agent improves the availability of both the flavan and the flavonoid of the oral compositions of various embodiments of the present invention to oral surfaces. The flavan and free-B-ring flavonoid tend to be lipophilic and the bioavailability-enhancing agent enhances tissue uptake and/or efficacy of the active at the oral surface, even in the relatively aqueous environment of the oral cavity. The bioavailability-enhancing agent is preferably at least one of a solubilizing agent and an efficacy-enhancing agent.

The free-B-ring flavonoid(s) and flavan(s) for use in the invention are both a type of flavonoid. Flavonoids are a class of compounds generally found in plants that have the general structural skeleton C6-C3-C6 pattern and include compounds such as flavones, flavans, flavonols, isoflavones, dihydroflavonols, flavonones, and derivatives thereof.

Free-B-ring flavonoids constitute a group of flavonoids that generally contain a 2,3-double bond and/or a 4-oxo group in the "C-ring" of the compound, as shown in the structure below, but generally have no substitute groups on the aromatic B-ring.

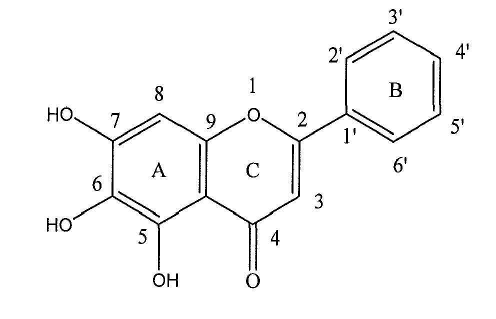

Flavans, which include flavonols, have a 2,3-double bond and generally lack a 4-oxo group (on the C-ring). Commonly occurring flavonol-type flavans have B-ring hydroxyl substituents; examples of these include flavan-3'-ols and flavan-3',4'-diols. Free-B-ring flavonoids can be isolated from a variety of different plant parts, including, but not limited to, stems, stem barks, trunks, trunk barks, twigs, tubers, roots, root barks, young shoots, tissues, seeds, rhizomes, flowers, and other reproductive organs, leaves and other aerial parts. For example, in various embodiments, free-B-ring flavonoids are isolated from plants of the taxa described in United States Patent Application Publication No. 2003/0216481 to Jia. All references cited in the present specification are hereby incorporated by reference in their respective entireties.

In various embodiments, free-B-ring flavonoids are isolated from plants of the family *Lamiaceae.* In various embodiments, the free-B-ring flavonoids are isolated from plants of the subfamily *Scutellarioideae.* In various embodiments, the free-B-ring flavonoids are isolated from plants of the genus *Scutellaria.* In various embodiments, the free-B-ring flavonoids are isolated from plants of the species *Scutellaria baicalensis.* The Chinese medicinal plant *Scutellaria baicalensis* contains significant amounts of free-B-ring flavonoids, including baicalein, baicalin, wogonin, and baicalenoside. Compositions comprising free-B-ring flavonoids have been shown to inhibit activity of the cyclooxygenase enzyme COX-2, *inter alia.*

Flavans are a specific class of flavonoids that can be isolated from a variety of different plant parts, including but not limited to stems, stem barks, trunks, trunk barks, twigs, tubers, roots, root barks, young shoots, tissues, seeds, rhizomes, flowers, and other reproductive organs, leaves and other aerial parts. In various embodiments, flavans can be isolated from plants of the taxa described in United States Patent Application Publication No. 2003/0216481 to Jia. In various embodiments, the flavans can be isolated from plants of the family *Fabaceae.* In various embodiments, the flavans can be isolated from plants of the subfamily *Mimosoideae.* In various embodiments, the flavans can be isolated from plants of the tribe *Acacieae.* In various embodiments, the flavans can be isolated from the genus *Acacia.* In various embodiments, the flavans can be isolated from the species *Acacia catechu.*

Catechin is an example of a flavan that is found extensively in *Acacia.* Catechin exhibits, both alone and in conjunction with flavonoids found in tea, antiviral and antioxidant activity. Catechin has also been shown to inhibit activity of both the COX-1 and COX-2 enzymes.

Free-B-ring flavonoids and flavans can be extracted from tissues of the above plants, and any other suitable plant, using any suitable extraction technique commonly known in the art. For example, extraction techniques that can be used are any suitable aqueous extraction or organic solvent extraction. Preferred extraction techniques utilize water, methanol, water/methanol, dichloromethane and methanol:THF. Any other suitable extraction technique may be used such as steam distillation, supercritical fluid extraction, and any of the techniques set forth in United States Patent Application Publication No. 2003/0216481 to Jia, for example.

The ratio of free-B-ring flavonoids to flavans in the oral composition can be adjusted based on a variety of different factors, such as the particular condition to be treated. In many applications, the ratio of free-B-ring flavonoids to flavans can be in the range of about 99:1 free-B-ring flavonoids: flavans to about 1:99 of free-B-ring flavonoids: flavans. Free-B-ring flavonoid-to-flavan ratios can be selected from the group consisting of approximately 90:10, 80:20, 70:30, 60:40, 50:50, 40:60, 30:70, 20:80, and 10:90. In various embodiments, the ratio of free-B-ring flavonoids: flavans is approximately 85:15. A variety of different techniques can be used to create the mixture of at least one free-B-ring flavonoid and at least one flavan. Specific examples of the preparation of this mixture are provided throughout United States Patent Application Publication No. 2003/0216481 to Jia.

The mixture of at least one free-B-ring flavonoid and at least one flavan can be present in the oral composition of the present invention at a variety of different amounts. In various embodiments the mixture can be present in the oral composition at more than about 0.001% by weight, of about 0.001% to about 5% by weight, of about 0.01% to about 5% by weight, of about 0.1% to about 3% by weight, of about 0.1 % to about 1% by weight, or of about 0.1 % to about 0.5% by weight.

At least one free-B-ring flavonoid and at least one flavan can be combined to form a mixture useful in a composition according to the present invention. Likewise, a plant extract containing at least one free-B-ring flavonoid can be combined with a plant extract containing at least one flavan to form a mixture for use therein. Such mixtures are also commercially available. An example is UNIVESTIN®, which is manufactured and sold by Unigen Pharmaceuticals, Inc., Superior, Colorado, United States. A full description of UNIVESTIN® can be found in United States Patent Application Publication No. 2003/0216481 to Jia. UNIVESTIN® is believed to inhibit specific enzymes that catalyze oral inflammatory pathways, such as, for example, the COX-1, COX-2, and 5-LO enzymes. In various embodiments the free-B-ring UNIVESTIN® flavonoids are isolated from plant tissues of the genus *Scutellaria.* Specific examples of methods useful for the preparation of a mixture of at least one free-B-ring flavonoid and at least one flavan are provided throughout United States Patent Application Publication No. 2003/0216481 to Jia, such as at Example 14.

In one embodiment, the mixture of at least one free-B-ring flavonoid and at least one flavan contains about 80 to about 90% by weight of active ingredient compounds, more specifically about 86% active compounds where 76% by weight are free-B-ring flavonoids and about 10% are flavans. In this mixture, the free-B-ring flavonoids are believed to include: baicalin (about 63%), wogonin-7-glucuronide (about 7%), oroxylin A 7-glucuronide (about 2%), baicalein (less than about 2%), wogonin (about 1 %), chrysin-7-glucouronide (about 1 %), 5-methyl-wogonin-7-glucuronide (about 0.5%), scutellarin (less than about 0.5%), norwogonin (less than about 0.5%), chrysin (less than about 0.2%), and oroxylin A (less than about 0.2%). The flavans are believed to include: catechin (about 10%) and epicatechin (less than about 0.5%).

In certain embodiments the bioavailability-enhancing agent comprises a solubilizing agent that serves to increase solubility of the flavan and/or flavonoid in the oral composition, which is particularly advantageous where the oral composition comprises a hydrophilic or aqueous carrier. In other embodiments, the bioavailability-enhancing agent comprises an efficacy-enhancing agent that improves or enhances the ability of the oral care active compound(s) to produce the desired effect on the target tissue. In certain embodiments, the bioavailability-enhancing agent comprises an efficacy-enhancing agent that comprises a delivery-enhancing group that enhances the delivery of the flavonoid and/or flavan (and optionally an oral care active agent) to oral tissue and/or a retention-enhancing group that increases retention of the flavonoid and/or flavan (and optionally an oral care active agent) by oral tissue. Optionally, the bioavailability-enhancing agent comprises both an efficacy-enhancing agent and a solubilizing agent.

As employed herein, the delivery-enhancing group refers to a moiety on the bioavailability-enhancing agent when it is an efficacy-enhancing agent, that attaches or substantively, adhesively, cohesively or otherwise bonds the efficacy-enhancing agent carrying components of the free-B-ring flavonoid/flavan mixture (and optionally the oral care active agents), to oral (*e.g*., tooth and gum) surfaces, thereby "delivering" the components and the optional oral care agent to the oral surfaces. The retention-enhancing group(s), which is generally hydrophobic, attaches or otherwise bonds free-B-ring flavonoids and/or flavans of the mixture, as well as the optional oral care agent (particularly where it is non-ionic) to the efficacy-enhancing agent, thereby promoting retention of these compounds directly on the efficacy-enhancing agent and indirectly on the oral surface(s). In some instances, attachment of the free-B-ring flavonoid/flavan mixture and optional oral care agent occurs through physical entrapment by the efficacy-enhancing agent, especially when the efficacy-enhancing agent is a cross-linked polymer and the structure inherently provides increased sites for such entrapment. The presence of a higher molecular weight, more hydrophobic cross-linking moiety in the cross-linked polymer still further promotes the physical entrapment of free-B-ring flavonoid(s), flavan(s), and the optional oral care agent in or on the cross-linked efficacy-enhancing agent polymer.

In certain embodiments, the bioavailability-enhancing agent comprises an efficacy-enhancing agent that comprises water soluble or swellable anionic polymer or co-polymer having delivery-enhancing groups and retention-enhancing groups, where the delivery-enhancing groups enhance delivery of free-B-ring flavonoids, flavans, and/or oral care agent(s) to a subject's oral tissue, and the retention-enhancing groups enhance retention by oral tissue of the free-B-ring flavonoids, flavans, and/or oral care agent(s).

The efficacy-enhancing agents can also include those that are characterized as having utility as denture adhesives or fixatives or dental cements. The efficacy-enhancing agent is a polymer or copolymer, which terms are entirely generic, thus including for example oligomers, homopolymers, copolymers of two or more monomers, ionomers, block copolymers, graft copolymers, cross-linked polymers and copolymers, and the like. They may be natural or synthetic, and water (saliva) soluble or swellable (hydratable, hydrogel forming) polymer or copolymer.

If the efficacy-enhancing agent comprises a delivery-enhancing group, it can be any of those listed in United States Patent Nos. 5,538,715 and 5,776,435 both to Gaffar et al. In various embodiments, the delivery-enhancing groups are preferably acidic such as sulfonic, phosphinic, or more preferably phosphonic or carboxylic, or a salt thereof, *e.g*., alkali metal or ammonium. When present, the retention-enhancing group(s) can be any organic retention-enhancing group, for example, those that have the formula -(X)ₙ -R wherein X is O, N, S, SO, SO₂, P, PO or Si or the like, R is hydrophobic alkyl, alkenyl, acyl, aryl, alkaryl, aralkyl, heterocyclic or their inert-substituted derivatives, and n is zero or one or more. The term "inert-substituted derivatives" is intended to include substituents on R which are generally non-hydrophilic and do not significantly interfere with the desired functions of the efficacy-enhancing agent as enhancing the delivery of the oral composition actives (including the flavonoid, flavan, and optionally oral care active(s)) to, and retention thereof, on oral surfaces such as halo, *e.g*., Cl, Br, I, and carboxy and the like.

The efficacy-enhancing agent can be selected to have various sizes. For example, in embodiments where the efficacy-enhancing agent comprises a synthetic anionic polymeric or linear anionic polymeric polycarboxylate, it can have an average M.W. of about 100 to about 5,000,000, 1,000 to about 3,000,000, 100,000 to about 3,000,000, and can be present in the oral composition at about 0.0005 to about 5% by weight, about 0.005 to about 4% by weight, or about 0.05 to about 3% by weight. Preferred copolymers are 1:4 to 4:1 copolymers of maleic anhydride or acid with another polymerizable ethylenically unsaturated monomer, preferably methyl vinyl ether (methoxyethylene), often referred to as PVM/MA. Examples of these copolymers are available from ISP Corporation under the trade name GANTREZ®, *e*.*g*., AN 139 (M.W. 1,100,000), AN 119 (M.W. 200,000); S-97 Pharmaceutical Grade (M.W. 1,500,000), AN 169 (M.W. 2,000,000), and AN 179 (M.W. 2,400,000); wherein the preferred copolymer is S-97 Pharmaceutical Grade (M.W. 1,500,000).

The delivery-enhancing groups of the efficacy-enhancing agent can also be various phosphonates. Such phosphonate-type efficacy-enhancing agents can have an average M.W. of about 100 to about 1,000,000 or about 1,000 to about 1,000,000. The efficacy-enhancing agent can be a polyvinyl phosphonate and/or alkali metal polyvinyl phosphonate and/or ammonium polyvinyl phosphonate of M.W. about 1000 or more. The phosphonate-type efficacy-enhancing agent can be present in the oral composition at about 0.0005 to about 4% by weight. The efficacy-enhancing agent can be a poly(β-styrenephosphonate), poly(α-styrenephosphonate), copoly(α,β-styrenephosphonate) or another copolymer of α-or β-styrenephosphonate with another polymerizable ethylenically unsaturated monomer, such as copoly (β-styrenephosphonate/vinylphosphonate). The phosphonate-type efficacy-enhancing agent can have an average M.W. of about 2,000 to about 30,000. In various embodiments, the efficacy-enhancing agent is present in the oral composition from about 0.0005 to about 5% by weight.

The oral composition comprises a bioavailability-enhancing agent that can, and preferably will, also include one or more solubilizing agents to solubilize the mixture of at least one free-B-ring flavonoid and at least one flavan. In certain embodiments, the solubilizing agent also solubilizes one or more oral care agent(s), particularly where the compounds are non-ionic or lipophilic. The solubilizing agent can be any solubilizing agent that is effective to solubilize the mixture of at least one free-B-ring flavonoid and at least one flavan. For example, in various embodiments the solubilizing agent can include one or more of the following: ethers, ketones, glycols (both polyethylene glycol (PEG) and methylated PEG), fats, oils, lipids, pyrrolidones, and polypyrrolidones. The solubilizing agent can further include dimethylsulfoxide (DMSO), dimethylformamide (DMF), N-methylpyrrolidone, and other polar aprotic solvents, such as polar aprotic solvents with a dielectric constant greater than 30. Also, the solubilizing agent can include an orally acceptable surface active agent, *e.g*., a surfactant, flavoring oil, alcohol, and solubilizing humectant (*e.g*., propylene glycol).

Examples of surfactants that can be used include anionic, nonionic, amphoteric, zwitterionic, and cationic synthetic detergents. Anionic surfactants include the water-soluble salts of alkyl sulfates having 8-20 carbon atoms in the alkyl radical (such as sodium alkyl sulfate), a monoalkyl phosphate compound having 6-18 carbon atoms, the water-soluble salts of sulfonated monoglycerides of fatty acids having from 8-20 carbon atoms (such as sodium lauryl sulfate (>82% pure) and sodium coconut monoglyceride sulfonates), an alkyl glycoside that is mono[alkyl(C₁₂-C₂₂)] - [(Glyc)₁₋₂₀], sarcosinates (such as sodium and potassium salts of lauroyl sarcosinate, myristoyl sarcosinate, palmitoyl sarcosinate, stearoyl sarcosinate and oleoyl sarcosinate), taurates, higher alkyl sulfoacetates (such as sodium lauryl sulfoacetate), isothionates (such as sodium lauroyl isothionate), sodium laureth carboxylate, sodium dodecyl benzenesulfonate, and mixtures of the foregoing. Preferred are the sarcosinates since they inhibit acid formation in the mouth due to carbohydrate breakdown.

Nonionic surfactants include poloxamers (sold under the tradename PLURONIC®); polyoxyethylene sorbitan esters (sold under the tradename TWEEN®); fatty alcohol ethoxylates; polyethylene oxide condensates of alkyl phenols; products derived from the condensation of ethylene oxide with fatty acids, fatty alcohols, fatty amides, or polyhydric alcohols; and polypropyleneoxide or ethylene oxide condensates of aliphatic alcohols; long-chain tertiary amine oxides; long-chain tertiary phospine oxides; long-chain dialkyl sulfoxides; and mixtures of such materials. Amphoteric surfactants include betaines (such as cocamidopropyl betaine), derivatives of aliphatic secondary and tertiary amines in which the aliphatic radical can be a straight or branched chain and wherein one of the aliphatic substituents contains about 8-18 carbon atoms and one contains an anionic water-solubilizing group (such as carboxylate, sulfonate, sulfate, phosphate or phosphonate), and mixtures of such materials.

Zwitterionic surfactants include derivatives of aliphatic quaternary ammonium, phosphonium and sulfonium compounds in which the aliphatic radical can be a straight or branched chain and wherein one of the aliphatic substituents contains about 8-18 carbon atoms and one contains an anionic water-solubilizing group (such as carboxy, sulfonate, sulfate, phosphate or phosphonate).

Cationic surfactants include aliphatic quaternary ammonium compounds having one long alkyl chain containing about 8-18 carbon atoms (such as lauryl trimethylammonium chloride, cetyl pyridinium chloride, cetyltrimethylammonium bromide, diisobuytylphenoxyethyldimethylbenzylammonium chloride, coconut alkyltrimethylammonium nitrite, cetyl pyridinium fluoride). Certain cationic surfactants can also act as antimicrobials.

The oral composition can also include humectant polyols and esters to assist in dissolving the mixture of free-B-ring flavonoid and flavan, optionally an oral care agent, to permit delivery to the soft oral tissues at or near the gum line. Any suitable humectant polyols and esters can be used, such as any one or more of: propylene glycol, dipropylene glycol and hexylene glycol; cellosolves such as methyl cellosolve and ethyl cellosolve; vegetable oils and waxes containing at least about 12 carbon atoms in a straight chain, such as olive oil, castor oil and glyceryl tristearate; and esters such as, amyl acetate, ethyl acetate, and benzyl benzoate. Petrolatum may also be used, as well as glycerine, sorbitol, and/or xylitol. Propylene glycol is preferred. As used herein, "propylene glycol" includes 1,2-propylene glycol and 1,3-propylene glycol. Propylene glycol can be present in any suitable amount, such as an amount sufficient to dissolve the antibacterial agent and/or the free-B-ring flavonoid/flavan mixture and prevent precipitation thereof upon dilution with saliva.

Any suitable flavoring or sweetening material may also be used as a solubilizing agent and to enhance the palatability of the oral composition. Examples of suitable flavoring constituents are flavoring oils, *e.g*., oil of spearmint, peppermint, wintergreen, sassafras, clove, sage, eucalyptus, marjoram, cinnamon, lemon, and orange, and methyl salicylate. Suitable sweetening agents include sucrose, lactose, maltose, xylitol, sodium cyclamate, sucralose, perillartine, AMP (aspartyl phenylalanine, methyl ester), saccharine and the like. Flavoring oil is believed to aid the dissolution of certain non-ionic active agent compounds. A phenolic flavor mixture comprising eucalyptol, thymol, methyl salicylate, and menthol can also be used.

The flavor and/or sweetening material can be present in any suitable amount. In various embodiments, the flavor and/or sweetening material can be present in an amount sufficient to dissolve the mixture of free-B-ring flavonoids, flavans, and the optional active agent and prevent precipitation thereof upon dilution with saliva. In various embodiments, the flavor material can be present at an amount of about 0.5% to about 50% by weight of a bioavailability-enhancing agent that is a solubilizing agent for the free-B-ring flavonoid/flavan mixture and in an amount sufficient to dissolve the free-B-ring flavonoid/flavan mixture in saliva. In various other embodiments, the flavor and/or sweetening material can be present in an amount effective to increase uptake of the free-B-ring flavonoid/flavan mixture by oral tissue. Thus, in certain embodiments, the flavoring and/or sweetening agents may each or together comprise from about 0.001 to about 5% of the oral composition. In certain embodiments, where the oral care agent comprises a non-ionic antibacterial agent, the flavor can be present at an amount of about 0.02% to about 2% phenolic flavor mix in an amount such that the ratio of a substantially water insoluble noncationic antibacterial agents: phenolic flavor is about 5:1 - 1:100.

The solubilizing agent(s) can be present in various amounts in the oral composition, such as an amount sufficient to dissolve the mixture of free-B-ring flavonoids and flavans and to prevent precipitation thereof upon dilution with saliva. The solubilizing agent(s) can also be present in an amount effective to increase the uptake of the antibacterial agent and the mixture of free-B-ring flavonoids and flavans by dental tissue. The solubilizing agent(s) are preferably present at about 0.02 to 50% by weight.

In various embodiments, the oral composition optionally comprises one or more oral care agents. In certain embodiments, the oral care agent is selected from the group consisting of: an antibacterial agent, an antimicrobial agent, an anti-caries agent, an anti-tartar agent, an anti-attachment agent, a biofilm disruption agent, an anti-inflammatory agent (in addition to the free-B-ring flavonoid and flavan), an antibiotic, and mixtures thereof. As appreciated by one of skill in the art, an oral care active compound or agent may fall into one or more of these classifications, as it may have multiple mechanisms and/or effects, and should not be construed as being limited to a single function or classification. In certain embodiments, the oral care active compound has a functionality or mechanism for preventing and/or treating an oral care disease, where the mechanism complements and/or supplements the mechanism provided by the free-B-ring flavonoid and flavan, as described above.

The oral composition optionally comprises an effective anti-plaque and/or anti-gingivitis amount of one or more oral care agents. Any suitable antibacterial or anti-plaque oral care agents can be used. Orally acceptable oral care agents among those useful herein include non-ionic antibacterial agents, cationic antibacterial agents, cationic active compounds, and anionic antibacterial agents.

In certain embodiments, the oral care agent is a non-ionic antibacterial agent that can include various phenolic compounds, including phenol and its homologs, mono- and poly- alkyl and aromatic halo- (*e.g*., fluorine, chlorine, bromine, iodine) - phenols, resorcinol and catechol and their derivatives and bisphenolic compounds. Such compounds include:

**Phenol and its Homologs**

| Phenol | |
|---|---|
| 2 Methyl | Phenol |
| 3 Methyl | Phenol |
| 4 Methyl | Phenol |
| 4 Ethyl | Phenol |
| 2,4-Dimethyl | Phenol |
| 2,5-Dimethyl | Phenol |
| 3,4-Dimethyl | Phenol |
| 2,6-Dimethyl | Phenol |
| 4-n Propyl | Phenol |
| 4-n-Butyl | Phenol |
| 4-n-Amyl | Phenol |
| 4-tert-Amyl | Phenol |
| 4-n-Hexyl | Phenol |
| 4-n-Heptyl | Phenol |
| 2-Methoxy-4-(2-Propenyl) | Phenol (Eugenol) |
| 2-Isopropyl-5-Methyl | Phenol (Thymol) |

| Mono- and Poly-Alkyl and Aralkyl Halophenols | |
|---|---|
| Methyl | p-Chlorophenol |
| Ethyl | p-Chlorophenol |
| n-Propyl | p-Chlorophenol |
| n-Butyl | p-Chlorophenol |
| n-Amyl | p-Chlorophenol |
| sec-Amyl | p-Chlorophenol |
| n-Hexyl | p-Chlorophenol |
| Cyclohexyl | p-Chlorophenol |
| n-Heptyl | p-Chlorophenol |
| n-Octyl | p-Chlorophenol |

| O-Chlorophenol | |
|---|---|
| Methyl | o-Chlorophenol |
| Ethyl | o-Chlorophenol |
| n-Propyl | o-Chlorophenol |
| n-Butyl | o-Chlorophenol |
| n-Amyl | o-Chlorophenol |
| tert-Amyl | o-Chlorophenol |
| n-Hexyl | o-Chlorophenol |
| n-Heptyl | o-Chlorophenol |

| p-Chlorophenol | |
|---|---|
| o-Benzyl | p-Chlorophenol |
| o-Benzyl-m-methyl | p-Chlorophenol |
| o-Benzyl-m, m-dimethyl | p-Chlorophenol |
| o-Phenylethyl | p-Chlorophenol |
| o-Phenylethyl-m-methyl | p-Chlorophenol |
| 3-Methyl | p-Chlorophenol |
| 3,5-Dimethyl | p-Chlorophenol |
| 6-Ethyl-3-methyl | p-Chlorophenol |
| 6-n-Propyl-3-methyl | p-Chlorophenol |
| 6-iso-propyl-3-methyl | p-Chlorophenol |
| 2-Ethyl-3,5-dimethyl | p-Chlorophenol |
| 6-sec Butyl-3-methyl | p-Chlorophenol |
| 2-iso-Propyl-3,5-dimethyl | p-Chlorophenol |
| 6-Diethylmethyl-3-methyl | p-Chlorophenol |
| 6-iso-Propyl-2-ethyl-3-methyl | p-Chlorophenol |
| 2-sec Amyl-3,5-dimethyl | p-Chlorophenol |
| 2-Diethylmethyl-3,5-dimethyl | p-Chlorophenol |
| 6-sec Octyl-3-methyl | p-Chlorophenol |

| p-Bromophenol | |
|---|---|
| Methyl | p-Bromophenol |
| Ethyl | p-Bromophenol |
| n-Propyl | p-Bromophenol |
| n-Butyl | p-Bromophenol |
| n-Amyl | p-Bromophenol |
| sec-Amyl | p-Bromophenol |
| n-Hexyl | p-Bromophenol |
| Cyclohexyl | p-Bromophenol |

| o-Bromophenol | |
|---|---|
| tert-Amyl | o-Bromophenol |
| n-Hexyl | o-Bromophenol |
| n-Propyl-m,m-Dimethyl | o-Bromophenol |
| 2-Phenyl Phenol | |
| 4-Chloro-2-methyl phenol | |
| 4-chloro-3-methyl phenol | |
| 4-chloro-3,5-dimethyl phenol | |
| 2,4-dichloro-3,5-dimethyl phenol | |
| 3,4,5,6-tetrabromo-2-methylphenol | |
| 5-methyl-2-pentylphenol | |
| 4-isopropyl-3-methylphenol | |
| 5-chloro-2-hydroxydiphenyl methane | |

| Resorcinol and Its Derivatives | |
|---|---|
| Resorcinol | |
| Methyl | Resorcinol |
| Ethyl | Resorcinol |
| n-Propyl | Resorcinol |
| n-Butyl | Resorcinol |
| n-Amyl | Resorcinol |
| n-Hexyl | Resorcinol |
| n-Heptyl | Resorcinol |
| n-Octyl | Resorcinol |
| n-Nonyl | Resorcinol |
| Phenyl | Resorcinol |
| Benzyl | Resorcinol |
| Phenylethyl | Resorcinol |
| Phenylpropyl | Resorcinol |
| p-Chlorobenzyl | Resorcinol |
| 5-Chloro | -2,4-Dihydroxydiphenyl Methane |
| 4'-Chloro | -2,4-Dihydroxydiphenyl Methane |
| 5-Bromo | -2,4-Dihydroxydiphenyl Methane |
| 4'-Bromo | -2,4-Dihydroxydiphenyl Methane |

| Bisphenolic Compounds |
|---|
| Bisphenol A |
| 2,2'-methylene bis (4-chlorophenol) |
| 2,2'-methylene bis (3,4,6-trichlorophenol) (hexachlorophene) |
| 2,2'-methylene bis (4-chloro-6-bromophenol) |
| bis (2-hydroxy-3,5-dichlorophenyl) sulfide |
| bis (2-hydroxy-5-chlorobenzyl) sulfide |

A further exemplary illustrative list of useful oral care antibacterial agents is provided in United States Patent Nos. 5,776,435 to Gaffar et al., 5,681,548 to Esposito et al., 5,912,274 and 5,723,500 both to Stringer et al.

In various embodiments, the non-ionic antibacterial agent comprises a phenolic and/or bisphenolic compounds, such as, halogenated diphenyl ethers, including triclosan (2,4,4'-trichloro-2'-hydroxy-diphenylether, triclocarban (3,4,4-trichlorocarbanilide), 2-phenoxyethanol, benzoate esters, carbanilides, phenols, thymol, eugenol, hexyl resorcinol and 2,2'-methylene bis (4-chloro-6-bromophenol). Such antibacterial agents may be present in various amounts, such as about 0.001 to about 5% by weight.

In some embodiments, the antibacterial agent can be a substantially water insoluble, non-ionic, antibacterial agent as discussed in United States Patent No. 5,292,526 to Gaffar et al. In certain embodiments, the non-ionic antibacterial agent comprises a halogenated diphenyl ether, preferably 2',4,4'-trichloro-2-hydroxy-diphenyl ether (triclosan). triclosan can be present in the oral composition in various amounts, such as an amount of about 0.01 % to about 5% by weight or about 0.25% to about 0.35% by weight of the oral composition.

The oral care agent may also optionally comprise a cationic antibacterial agent. Suitable cationic antibacterial agents for use in oral compositions include, for example:
(i) quaternary ammonium compounds, such as those in which one or two of the substituents on the quaternary nitrogen has from 8 to 20, preferably from 10 to 18 carbon atoms and is preferably an alkyl group, which may optionally be interrupted by an amide, ester, oxygen, sulfur, or heterocyclic ring, while the remaining substituents have a lower number of carbon atoms, for instance from 1 to 7, and are preferably alkyl, for instance methyl or ethyl, or benzyl. Examples of such compounds include benzalkonium chloride, dodecyl trimethyl ammonium chloride, benzyl dimethyl stearyl ammonium chloride, hexadecyltrimethyl ammonium bromide, benzethonium chloride (diisobutyl phenoxyethoxyethyl dimethyl benzyl ammonium chloride) and methyl benzethonium chloride;
(ii) pyridinium and isoquinolinium compounds, including hexadecylpyridinium chloride, alkyl isoquinolinium bromides; tetradecylpyridinium chloride, and N-tetradecyl-4-ethylpyridinium chloride;
(iii) pyrimidine derivatives such as hexetidine (5-amino-1,3-bis(2-ethylhexyl)-5-methyl-hexahydropyrimidine);
(iv) amidine derivatives such as hexamidine isethionate (4,4'-diamidino-α ω-diphenoxy-hexane isethionate);
(v) bispyridine derivatives such as octenidine dihydrochloride (N,N' [1,10-decanediyldi-1 (4H)-pyridinyl-4-ylidene]-bis (1-octanamine) dihydrochloride);
(vi) guanides, for example, mono-biguanides such as p-chlorobenzyl-biguanide and N'(4-chlorobenzyl)-N"-(2,4-dichlorobenzyl) biguanide, poly(biguanides) such as polyhexamethylene biguanide hydrochloride, and bis-biguanides of the general formula (1): 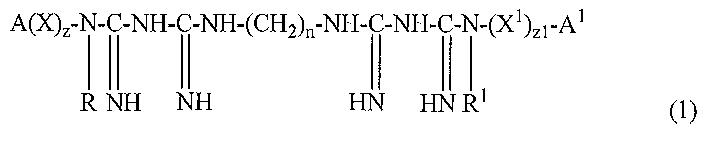
(vii) in which A and A¹ each represent (i) a phenyl group optionally substituted by (C₁₋₄) alkyl, (C₁₋₄) alkoxy, nitro, or halogen, (ii) a (C₁₋₁₂) alkyl group, or (iii) a (C₄₋₁₂) acyclic group; X and X¹ each represent (C₁₋₃) alkylene; R and R¹ each represent hydrogen, (C₁₋₁₂) alkyl, or aryl (C₁₋₆) alkyl; Z and Z1 are each 0 or 1; n is an integer from 2 to 12; and the polymethylene chain (CH₂)ₙ may optionally be interrupted by oxygen or sulfur or an aromatic (for instance, phenyl or naphthyl) nucleus; and orally acceptable acid addition salts thereof; examples of such bis-biguanides include chlorhexidine and alexidine. Suitable acid addition salts of the bis-biguanides of general formula (1) include the diacetate, the dihydrochloride and the digluconate. Suitable acid addition salts of chlorhexidine include the digluconate, diformate, diacetate, dipropionate, dihydrochloride, dihydroiodide, dilactate, dinitrate, sulfate, and tartrate salts. Suitable acid addition salts of alexidine include the dihydrofluoride and the dihydrochloride salts; and

Other optional oral care agents that are cationic compounds include N^{α}-acyl amino acid alkyl esters and salts generally represented by the formula (2) below: 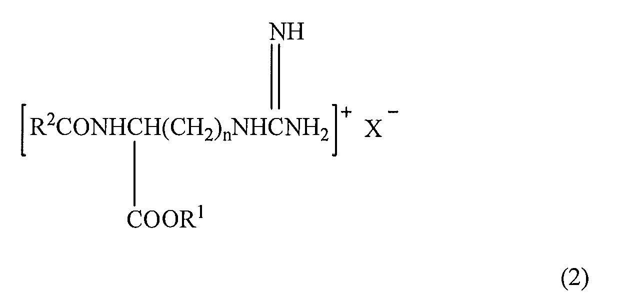 where R¹ is an alkyl chain of 1 to 8 carbon atoms, preferably from 1 to 3 carbon atoms, and most preferably 3 carbon atoms; R² is an alkyl chain of 6 to 30 carbon atoms, preferably from 10 to 12 carbon atoms, and mixtures thereof; and X is an anion. In various embodiments, the R²CO moiety comprises a natural fatty acid residue such as a natural fatty acid selected from the group consisting of coconut oil fatty acid, tallow fatty acid residue, or a mono-fatty acid residue such as selected from the group consisting of lauroyl (C₁₂), myristyl (C₁₄), stearoyl (C₁₈) fatty acid residues, and mixtures thereof. The R²CO moiety comprises a lauroyl fatty acid residue in certain embodiments.

X may be any counter-anion that provides a reasonable degree of solubility in water (preferably at least about 1g in 1L of water). Examples of X counter anions which form ester salts of the above identified formula, include inorganic acid salts, such as those comprising halogen atoms (*e.g*., chloride or bromide) or dihydrogen phosphate, or an organic salt such as acetate, tautarate, citrate, or pyrrolidonecarboxylate (PCA). The chloride salt is preferred.

Examples of esters of the above-identified formula wherein n in the formula equals 3 useful for the present oral compositions include N^{α}-cocoyl-L-arginine methyl ester, N^{α}-cocoyl-L-arginine ethyl ester, N^{α}-cocoyl-L-arginine propyl ester, N^{α}-stearoyl-L-arginine methyl ester, N^{α}-stearoyl-L-arginine ethyl ester salts, such as hydrochloride. In one embodiment, the cationic oral care agent comprises a hydrogen chloride salt of ethyl lauroyl arginine (ELAH).

Thus, in certain embodiments, preferred cationic active ingredients are selected from the group consisting of benzethonium chloride, octenidine, hexetidine, hexamidine, cetyl pyridinium chloride, chlorhexidine, alexidine, N^{α}-acyl amino acid alkyl ester salts, and mixtures thereof. In certain embodiments, a cationic oral care agent comprises cetyl pyridinium chloride (CPC). In certain embodiments, the oral care agent comprises an N^{α}-acyl amino acid alkyl ester salt, such as ethyl lauroyl arginine ester hydrochloride (ELAH).

In other embodiments, an oral care active agent is an anti-attachment agent. While not limiting as to the present invention, oral care active ingredients are generally believed to operate by either (or both) of two predominant anti-attachment mechanisms. Biofilms (also referred to as pellicle) are a matrix formed on an oral surface, typically on a hard tissue surface, comprising bacteria (generally about 60-70% of the biomatrix), bacterial extracellular byproducts, proteins, lipids, and glycolipids.

Early stages of biofilm formation include attachment of an initial bacteria layer to an oral surface, generally believed to be attached by ligands or adhesins on the bacterial cell wall that interact with receptors on an oral surface. It is believed that the bacterial cells attach to the salivary glycoproteins on the oral surface, *e.g*., enamel. The bacteria appear to form a stronger attachment by generating extracellular glucan polymers to adhere to the oral surface. The bacteria then grow and divide forming a dense layer on the oral surface. After a specific density is reached, it is believed that a variety of species of bacteria attach to the anchoring layer of bacteria to form the biofilm matrix.

Thus, anti-attachment agents can interact with an oral surface, such that the bacteria and biofilm components cannot adhere to the oral surface and no anchoring layer can be formed on the oral surface. Such an anti-attachment agent may substantially cover an oral surface, and prevent attachment of the bacteria and other components of the biofilm matrix. A second mechanism is one where the anti-attachment agent interacts with the bacteria itself to disable it from attaching to the oral surface, likely by interacting with the adhesins, ligands, or other moieties on the surface of the bacteria that would ordinarily facilitate a linkage with a receptor or other moiety at the oral surface.

While not limiting as to the present invention, it is believed that in some embodiments the N^{α}-acyl amino acid alkyl ester salts described above, such as ethyl lauroyl arginate hydrochloride (ELAH), function as an anti-attachment active ingredient. While not limiting as to the mechanism by which such oral care agents function, ELAH appears to alter the tooth surface energy (reducing it) to prevent adherence and attachment of microorganisms that may form a plaque biofilm on the tooth surface. ELAH appears to have substantivity on the tooth surface, such that it remains attached for a sufficient period of time to effectively prevent microorganisms from adhering to the tooth surface, thereby preventing or reducing biofilm formation.

In accordance with various embodiments of the present invention, the application of the ELAH as an active ingredient promotes longer and more effective anti-plaque benefits at lower concentrations in comparison with many antimicrobial ingredients which are washed away in the aqueous oral cavity.

In some embodiments, the oral care agent comprises an oral care active that is a biofilm disruption agent. A biofilm disruption agent is generally a compound that prevents formation of and/or attacks a biofilm (or pellicle) already formed on an oral surface.

Enzymes have conventionally been selected as biofilm disruption agents, based on an ability to hydrolyze proteins, starch and lipids, which form a part of a biofilm matrix. In certain embodiments, such enzymes include, by way of example, protease enzymes, such as cysteine proteases or serine proteases. Examples are most desirably selected from the group: papain (for example, isolated from the latex of the green fruit and leaves of *Carica papaya*), ficin (for example, isolated from the latex of tropical fig trees *Ficus glabrata*), krillase (for example, isolated from Antarctic krill), other cysteine and serine proteases, glucoamylase, dextranase, mutanase, lysozyme, plant lipase, gastric lipase, pancreatic lipase, tannase, bromelain, chymotrypsin, alcalase, amalysecs, lactoferrin, gingipains, glucose oxidase, elastases and/or cellusases pectinase, and mixtures thereof. Other exemplary biofilm disruption agents for the oral cavity include synthetic histatin, furanone, derivatives of furanone, and mixtures of any of the above.

The oral compositions of the present invention can optionally comprise other anti-plaque/plaque disrupting agents in addition to those set forth above, including without limitation: copper, magnesium, and strontium salts; dimethicone copolyols such as cetyl dimethicone copolyol; urea; calcium lactate; calcium glycerophosphate; strontium polyacrylates; and mixtures thereof.

In certain embodiments, the oral care agent comprises an oral care active compound that is an anti-inflammatory agent. Inflammation of the oral tissue generally refers to a localized protective response elicited by injury or destruction of tissues, which serves to destroy, dilute, or sequester both the injurious agent and the injured tissue. Chronic inflammation is often a continuation of acute inflammation and is a prolonged low-grade form of inflammation (such as that associated with periodontitis or gingivitis) and usually causes permanent tissue damage. Histologically, inflammation involves a complex series of events and corresponds to enhanced levels of pro-inflammatory cellular mediators (substances that are released from cells) as the result of the interaction of an antigen with an antibody or by the action of antigen with a sensitized lymphocyte.

The suppression of one or more of the above described pro-inflammatory mediators prevents and/or treats tissue damage and/or tissue loss associated with oral disease. Thus, oral compositions that comprise an additional anti-inflammatory agent (in addition to the free-B-ring flavonoid and the flavan) to suppress one or more mediators of inflammation are useful for the certain embodiments of the present invention. The anti-inflammatory agent can also suppress immune system recognition of one or more antigens produced by pathogens in an oral cavity. For example, gram-negative bacteria have endotoxins, generally known as lipopolysaccharide (LPS) components that are antigens detected by various cells within the immune system to produce an immune system response that includes production of inflammatory mediators and activation of the cascade complement and coagulation cascade. Certain useful anti-inflammatory compounds in accordance with the present invention prevent a subject's immune system from recognizing or responding to one or more antigens present in the oral cavity, such as LPS on the cell walls of gram-negative bacteria. Such anti-inflammatory drugs are believed to interface with antigens in such a manner that the microbe/bacteria/antigen is no longer recognized by the receptors of certain cells of the immune system either by effectively cloaking it from immune system detection or preventing an immune system response through suppression of intercellular mediators generated in response to recognition of LPS, thereby suppressing an immune system response. For example, LPS is believed to trigger a complex cascade of immune system events, including for example, the activation and release of NF-κB (Nuclear Factor kappa B). NF-kB is believed to trigger immune response, including osteolysis and osteomyelitis, in response to LPS generated by bacteria in the oral cavity. Compounds that regulate or prevent NF-kB production are useful for the present invention. Such examples include parthenolides, such as sesquiterpene lactone parthenolides, which are believed to inhibit LPS-induced osteolysis. Other non-limiting examples of such active agent compounds include androstenediol (AED) and dehydroepiandrosterone (DHEA). The present invention further contemplates other such active compounds that have been or will be discovered for such purposes.

Other useful anti-inflammatory agents can include those that prevent the accumulation of inflammatory mediators, such as those derived from arachidonic acid pathway, that are triggered by immune system detection of an antigen. One class of mediators that modulate inflammatory response are arachidonic acid metabolites, namely prostaglandins, leukotrienes, and thromboxanes, which are produced through the cyclooxygenase or lipoxygenase enzyme pathways. These metabolites have been implicated as the prime mediators in gingivitis, periodontitis, osteomyelitis and other inflammatory diseases. By way of example, such anti-inflammatory agents that prevent the accumulation of inflammatory mediators from the arachidonic acid pathway, include non-steroidal anti-inflammatory drugs (NSAIDs). Examples of useful NSAID anti-inflammatory agents include those selected from the group consisting of: indomethicin, flurbiprofen, ketoprofen, ibuprofen, naproxen, meclofenamic acid, and mixtures thereof.

Likewise, another mechanism for oral care anti-inflammatory agents is one where the anti-inflammatory agent serves to reduce or scavenge one or more reactive oxide species within the oral cavity. Reactive oxygen species (ROS) are typically highly reactive products produced during various biochemical processes, and include superoxide anions (O₂⁻), hydrogen peroxide (H₂O₂), and hydroxyl radicals (·OH). The formation of ROS can occur as part of many cellular processes including immune cell responses, and cell injury. Increased ROS formation under pathological conditions is believed to cause cellular damage through the action of these highly reactive molecules by cross-linking proteins, mutagenizing DNA, and peroxidizing lipids. While not limiting to any theory by which the present invention is bound, it is generally believed that one mechanism by which the at least one free-B-ring flavonoid and the flavan operate is by reducing one or more ROS in the oral cavity, in addition to inhibiting specific enzymes that catalyze oral inflammatory pathways, such as and for example, the COX-1, COX-2, and 5-LO enzymes.

Other suitable anti-inflammatory agents useful for oral care active agents include oregano extract (for example, extracts from *Origanum vulgare* (commonly known as "oregano", "wild oregano", or "wild maijoram")) as disclosed in United States Patent Application Serial No. 11/256,788 to Worrell et al., filed October 24, 2005, or magnolia extract, derived from plants in the *Magnoliaceae* family, such as *Magnolia Officinalis* as described in United States Patent Application Serial No. 11/285,809 to Gaffar et al., filed November 23, 2005.

Optional exemplary antioxidants are butylated hydroxyanisole (BHA), butylated hydroxytoluene (BHT), vitamin A, carotenoids, tocopherols (vitamin E), flavonoids, polyphenols, ascorbic acid (vitamin C), herbal antioxidants, chlorophyll, melatonin, chloride, calcium, calcium oxide, calcium chloride, disodium ubiquinone (coenzyme Q₁₀), ethylhexyl gallate, hydrogen peroxide, iodine, lycopene, magnesium ascorbate, potassium sulfite, sodium bisulfite, thiolactic acid, and mixtures thereof.

In certain embodiments, the oral compositions comprise an oral care active agent that is an antibiotic, such as augmentin, amoxicillin, tetracycline, doxycycline, minocycline, metronidazole, neomycin, kanamycin and clindamycin; and mixtures thereof.

Additional optional oral care compounds that can be included as active ingredients in the oral composition include, for example, additional antibacterial agents not already discussed above, whitening agents, additional anti-caries and tartar control agents not already discussed above, periodontal actives, abrasives, breath freshening agents, malodour control agents, tooth desensitizers, salivary stimulants, whitening agents, analgesics, and combinations thereof. It is understood that while general attributes of each of the above categories of actives may differ, there may be some common attributes and any given material may serve multiple purposes within two or more of such categories of actives.

Exemplary actives among those useful herein are disclosed in United States Patent Nos. 4,894,220 to Nabi et al., 5,288,480 to Gaffar et al., United States Patent Application Publication No. 2003/0206874 to Doyle et al., as well as in United States Patent No. 6,290,933 to Durga et al., and United States Patent No. 6,685,921 to Lawlor. Such active ingredients are well known to one of skill in the art. Preferably, such actives are selected for compatibility with the free-B-ring flavonoid/flavan mixture. Further mixtures of oral care agents, even within the same classification, are contemplated by the present invention.

Active oral care agents useful herein are optionally present in the compositions of the present invention in safe and effective amounts. A "safe and effective" amount of an active is an amount that is sufficient to have the desired therapeutic or prophylactic effect in the human or lower animal subject to whom the active is administered, without undue adverse side effects (such as toxicity, irritation, or allergic response), commensurate with a reasonable benefit/risk ratio when used in the manner of this invention. The specific safe and effective amount of the active will vary with such factors as the particular condition being treated, the physical condition of the subject, the nature of concurrent therapy (if any), the specific active used, the specific dosage form, the carrier employed, and the desired dosage regimen. Oral care agents can be present in the oral care composition at quantities of from about 0.001 to about 5% by weight of the oral composition, unless otherwise specified below.

Any suitable fluoride ion source can be present in the oral composition, such as those recited in United States Patent No. 5,080,887 to Gaffar et al. A fluoride ion source may be slightly or fully water-soluble and typically has an anti-caries function. A fluoride ion source (or any ion source) is characterized by its ability to release fluoride ions in water and by freedom from undesired reaction with other compounds of the oral composition. One or more such sources can be present. Examples of such sources are inorganic metal and/or ammonium fluoride salts and compounds, such as, for example: sodium fluoride, potassium fluoride, ammonium fluoride, calcium fluoride, cuprous fluoride, zinc fluoride, barium fluoride, sodium silica fluoride, ammonium fluorosilicate, sodium fluorozirconate, sodium monofluorophosphate, stannous fluoride, aluminum mono- and di-fluorophosphate, and fluorinated sodium calcium pyrophosphate. The fluoride source can also be an amine fluoride, such as olaflur (N'octadecyltrimethylendiamine-N,N,N'-tris(2-ethanol)-dihydrofluoride). In some embodiments, sodium fluoride or sodium monofluorophosphate are preferred.

The amount of fluoride ion providing source is dependent to some extent upon the type of source, its solubility, and the form of the oral composition, but it will be present in a non-toxic amount, generally of about 0.001% to about 3.0% in the oral composition. In a dentifrice composition, for example, a dental gel, toothpaste (including cream), toothpowder, or dental tablet, an amount of such source which releases up to about 5,000 ppm of F⁻ ion by weight of the preparation is considered satisfactory. Any suitable minimum amount of such source may be used, but it is preferable to employ an amount sufficient to release about 300 to 2,000 ppm, more preferably about 800 to about 1,500 ppm of fluoride ion.

In various embodiments, the oral composition comprises an oral care agent that comprises one or more stannous ion sources, which are helpful for reducing gingivitis, plaque, calculus, caries and/or sensitivity, for example. One or more such sources can be present. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of about 0.01 % to about 10%, for example about 0.1 % to about 7% or about 1% to about 5% by weight of the composition.

In certain embodiments of the oral compositions, the combination of the at least one flavonoid, at least one flavan and an oral care active agent comprising a stannous ion source provides superior anti-gingivitis efficacy. Further, the combination of the at least one free-B-ring flavonoid and the at least one flavan with the stannous ion source appears to provide improved aesthetics of the oral composition, including improved color stability. In one example, the oral composition comprises UNIVESTIN® at about 0.5%, about 0.45% stannous fluoride, and about 0.6% stannous chloride.

In various embodiments, the oral composition comprises an oral care agent that comprises one or more zinc ion sources, which can be useful, for example, as antimicrobial, anticalculus or breath-freshening agents. Suitable zinc ion sources include without limitation zinc acetate, zinc chlorite, zinc citrate, zinc gluconate, zinc glycinate, zinc oxide, zinc sulfate, sodium zinc citrate and the like. One or more zinc ion sources are optionally and illustratively present in a total amount of about 0.05% to about 3%, for example about 0.1 % to about 1%, by weight of the composition.

The oral compositions of the present invention optionally comprise a saliva stimulating agent, useful for example in amelioration of dry mouth. Any orally acceptable saliva stimulating agent can be used, including without limitation, food acids such as citric, lactic, maleic, succinic, ascorbic, adipic, fumaric and tartaric acids, and mixtures thereof. One or more saliva stimulating agents are optionally present in a saliva stimulating effective total amount.

The compositions of the present invention optionally comprise an H₂ histamine receptor antagonist. H2 antagonists useful herein include cimetidine, etintidine, ranitidine, ICIA-5165, tiotidine, ORF-17578, lupititidine, donetidine, famotidine, roxatidine, pifatidine, lamtidine, BL-6548, BMY-25271, zaltidine, nizatidine, mifentidine, BMY-52368, SKF-94482, BL-6341A, ICI-162846, ramixotidine, Wy-45727, SR-58042, BMY-25405, loxtidine, DA-4634, bisfentidine, sufotidine, ebrotidine, HE-30-256, D-16637, FRG-8813, FRG-8701, impromidine, L-643728, HB-408.4, and mixtures thereof.

The compositions of the present invention optionally comprise a desensitizing agent. Desensitizing agents useful herein include potassium citrate, potassium chloride, potassium tartrate, potassium bicarbonate, potassium oxalate, potassium nitrate, strontium salts, and mixtures thereof. Alternatively or in addition, a local or systemic analgesic such as aspirin, codeine, acetaminophen, sodium salicylate or triethanolamine salicylate can be used.

The oral compositions optionally comprise a nutrient. Suitable nutrients include vitamins, minerals, amino acids, and mixtures thereof. Vitamins include Vitamins C and D, thiamine, riboflavin, calcium pantothenate, niacin, folic acid, nicotinamide, pyridoxine, cyanocobalamin, para-aminobenzoic acid, bioflavonoids, and mixtures thereof. Nutritional supplements include amino acids (such as L-tryptophane, L-lysine, methionine, threonine, levocarnitine and L-carnitine), lipotropics (such as choline, inositol, betaine, and linoleic acid), fish oil (including components thereof such as omega-3 (N-3) polyunsaturated fatty acids, eicosapentaenoic acid and docosahexaenoic acid), and mixtures thereof.

The oral composition can also include at least one anticalculus active agent, such as one or more of the anticalculus agents recited in United States Patent No. 5,292,526 to Gaffar et al. In various embodiments, the composition includes one or more anticalculus polyphosphates. The oral composition can include at least one wholly or partially neutralized alkali metal or ammonium tripolyphosphate or hexametaphosphate salt present in the oral composition at an effective anticalculus amount. The oral composition can also include at least one water soluble, linear, molecularly dehydrated polyphosphate salt effective in an anticalculus amount. The oral composition can also include a mixture of potassium and sodium salts at least one of which is present in an effective anticalculus amount as a polyphosphate anticalculus agent.

The anticalculus composition can also contain an effective anticalculus amount of linear molecularly dehydrated polyphosphate salt anticalculus agent present in a mixture of sodium and potassium salts. The ratio of potassium to sodium in the composition can be in the range of about 3:1, for example. The polyphosphate can be present in the oral composition in various amounts. An example of an oral composition comprises an oral care active agent, where the weight ratio of polyphosphate ion to an oral care agent ranges from in excess of about 0.7:1 to less than about 4:1, or wherein the weight ratio of the efficacy-enhancing agent to the polyphosphate ion ranges from about 1:6 to about 2.7:1, or wherein the weight ratio of the efficacy-enhancing agent to the polyphosphate ranges from about 1:6 to about 2.7:1. Other useful anticalculus agents include polycarboxylate polymers and polyvinyl methyl ether/maleic anhydride (PVME/MA) copolymers, such as GANTREZ®, discussed above in the context of a bioavailability-enhancing agent.

In order to optimize the anticalculus effectiveness of the oral composition, inhibitors against enzymatic hydrolysis of the polyphosphate are desirably present. Such agents are a fluoride ion source sufficient to supply 25 ppm to 5,000 ppm or 25 ppm to 2,000 ppm of fluoride ions at an amount of about 0.01% to about 5% by weight, and 0% to 3% of a synthetic anionic polymeric polycarboxylate having a molecular weight of about 1,000 to about 1,000,000, preferably about 30,000 to about 500,000.

When the oral composition is made by initially dissolving a polyphosphate and an optional oral care antibacterial agent in a humectant and surface active agent and incrementally adding the bioavailability-enhancing agent to the resulting oral composition, especially where the efficacy-enhancing agent is a polymeric polycarboxylate such as GANTREZ®, the solution becomes clear and may be characterized as a "microemulsion." As the amount of bioavailability-enhancing agent therein increases such that the complete oral composition contains at least about 2.2% by weight thereof, the solution becomes cloudy and may be characterized as a "macroemulsion." In such "macroemulsion" type compositions, the anti-plaque effect of the optional antibacterial agent appears to be optimized.

The compositions of the present invention optionally comprise an abrasive. In various embodiments, an abrasive is useful for example as a polishing agent. Any orally acceptable abrasive can be used, but type, fineness (particle size) and amount of abrasive should be selected so that tooth enamel is not excessively abraded in normal use of the composition. Suitable abrasives include silica, for example in the form of silica gel, hydrated silica or precipitated silica, alumina, insoluble phosphates, calcium carbonate, resinous abrasives such as urea-formaldehyde condensation products, and mixtures thereof. Among insoluble phosphates useful as abrasives are water-insoluble orthophosphates, polymetaphosphates and pyrophosphates. Illustrative examples of these include dicalcium orthophosphate dihydrate, calcium pyrophosphate, β-calcium pyrophosphate, tricalcium phosphate, calcium polymetaphosphate, and insoluble sodium polymetaphosphate. One or more abrasives are optionally present in an abrasive-effective total amount, typically about 5% to about 70%, for example about 10% to about 50% or about 15% to about 30% by weight of the composition. Average particle size of an abrasive, if present, is generally about 0.1 µm to about 30 µm, for example about 1 µm to about 20 µm, or about 5 µm to about 15 µm.

In certain embodiments, the oral care active agent of the oral composition comprises any of the following exemplary additional oral care active agents not previously discussed above, including α-ionone, grapeseed extract; thymol, eugenol, menthol, geraniol, carvacrol, citral, eucalyptol, 8-hydroxyquinoline and salts, copper (II) compounds such as copper (II) chloride, fluoride, sulfate and hydroxide, phthalic acid and salts thereof such as magnesium monopotassium phthalate, sanguinarine, salicylanilide, halogenated salicylanilides, domiphen bromide, sulfonamides, and piperidino derivatives such as delmopinol and octapinol.

The oral compositions comprise an orally acceptable vehicle or carrier. The carrier can be a liquid, semi-solid, or solid phase, in the form of a mouth rinse, dentifrice (including toothpastes, toothpowders, and prophylaxis pastes), confectionaries (including lozenges and gum), medicament, film, or any other form known to one of skill in the art. Selection of specific carrier components is dependant on the desired product form.

The oral composition of the present invention can be made by any of the methods known in the art for combining ingredients to make oral care compositions. Examples of methods that can be used are set forth in: United States Patent No. 6,403,059 to Martin et al.; Clinical Pharmacology for Dental Professionals (Mosby-Year Book, Inc., 3rd ed. 1989); Mosby's Dental Hygiene: Concepts, Cases and Competencies, (Daniel, Susan J., Harfst, and Sherry A. eds., Elsevier Science Health Science Div. 2002); and Ernest W. Flick, Cosmetic and Toiletry Formulations, 2nd ed.).

Conventional ingredients that can be used to form the carriers listed above are well known to the skilled artisan. Any suitable orally acceptable vehicle can be used, such as those described in United States Patent No. 4,894,220 to Nabi et al. As recognized by one of skill in the art, the oral compositions optionally include other materials in addition to those components previously described, including for example, surface active agents, such as surfactants, emulsifiers, and foam modulators, viscosity modifiers and thickeners, humectants, diluents, additional pH modifying agents, emollients, moisturizers, mouth feel agents, sweetening agents, flavor agents, colorants, preservatives, solvents, such as water, and combinations thereof. It is understood that while general attributes of each of the above categories of materials may differ, there may be some common attributes and any given material may serve multiple purposes within two or more of such categories of materials. Preferably, such carrier materials are selected for compatibility and stability with all of the constituents of the active ingredient, including free-B-ring flavonoid(s) and flavan(s) and the optional one or more oral care active agent compounds selected for the oral composition. Further, as described previously above, the carrier ingredient may also serve as a bioavailability-enhancing agent, either as an efficacy-enhancing agent or a solubilizing agent for the active ingredients.

Typical useful surface active agents are disclosed above in the context of the bioavailability-enhancing agent that includes a solubilizing agent. Surface active agents generally are an important aspect of the oral composition, as they can function as surfactants, emulsifiers, foam modulators, and/or active ingredient dispersion agents. Their selection for compatibility with the active ingredient constituents is important. For example, in embodiments where the oral composition has an active ingredient comprising a cationic active oral care agent, it is preferred that the carrier comprises surfactants that are not strongly anionic, as such anionic compounds can bind to the cationic active ingredient potentially reducing its bioavailability. Suitable surface active agents, include those that were discussed in the context of the bioavailability/solubility enhancing agent above, are those which are reasonably stable and foam throughout a wide pH range. In certain embodiments, one or more surface active agents are present in the oral composition in the range from about 0.001% to about 5%, preferably from about 0.5% to about 2.5%.

The oral composition can also include a thickening agent. Any suitable thickening agent well known to those of skill in the art can be used, such as those disclosed in United States Patent Nos. 6,692,726 to Morgan et al. and 6,696,047 to Scott et al. One or more thickening agents are optionally present in a total amount of about 0.01% to about 15%, for example, about 0.1% to about 10% or about 0.2% to about 5% by weight of the oral composition

In embodiments where the oral composition is in the form of a mouthrinse, an exemplary carrier is substantially liquid. The term "mouthrinse" includes washes, sprays, rinses, irrigants, and the like. In such a preparation the orally acceptable carrier typically has an aqueous phase comprising either water, or a water and alcohol mixture. Further, in various embodiments, the oral carrier typically has a humectant, surfactant, a pH buffering agent, and a sweetening and/or flavoring agent (such as those described previously above).

For example, a mouthrinse vehicle can be a water-alcohol mixture. Generally, the weight ratio of water to alcohol is in the range of from about 1:1 to about 20:1, preferably about 3:1 to 10:1 and more preferably about 4:1 to about 6:1. The total amount of water-alcohol mixture in, for example, a mouthwash is typically in the range of from about 70 to about 99.9% by weight. The alcohol is preferably non-toxic, such as ethanol or isopropanol. A humectant, such as glycerine, sorbitol, or xylitol may be present in an amount of about 10-30% by weight. The oral composition may contain water at about 5% to about 30% by weight. Liquid oral compositions typically contain about 50-85% of water, may contain about 0.5-20% by weight of alcohol and may also contain about 10-40% by weight of humectant, such as glycerine, sorbitol, and/or xylitol. Some materials are commercially available in aqueous solutions, such as sorbitol that is provided in 70% aqueous solution. Ethanol is one preferred non-toxic alcohol. It is believed that alcohol assists in dissolving the water-insoluble oral care agents, as well as the mixture of free-B-ring flavonoids and flavans, in essence performing as a solubilizing agent.

In embodiments where an oral composition is in the form of a confectionary, an exemplary carrier is substantially solid or semi-solid. Confectionary carriers are well known in the art. For a lozenge, the carrier typically comprises a lozenge base material (for example, comprising a non-cariogenic polyol and/or starch/sugar derivative), an emulsifier, a lubricant, a flavoring agent, a thickener, and optionally a coating material. Chewing gum carriers generally have a chewing gum base, one or more plasticizing agents, a sweetening agent, and a flavoring agent.

In embodiments where an oral composition is in the form of a film, an exemplary carrier is substantially solid or semi-solid. Generally, such film carriers comprise a water soluble or dispersible film forming agent, such as a hydrophilic polymer. Optionally, the film carrier may also comprise hydrophobic film forming polymers, either as a removable backing layer, or mixed with a hydrophilic film forming polymer. Film carriers optionally comprise plasticizers, surface active agents, fillers, bulking agents, and viscosity modifying agents.

In embodiments where an oral composition is in the form of a dentifrice, an exemplary carrier is substantially semi-solid or a solid. Dentifrices typically contain surface active agents, humectants, viscosity modifying agents and/or thickeners, abrasives, solvents, such as water, flavoring agents, and sweetening agents.

In one example of a dentifrice, the carrier comprises a water-phase and humectant. For certain dentifrices, the water and humectant liquid phase comprise at least about 10% by weight of the oral composition. Moreover, preferably a humectant comprises propylene glycol, which serves as a bioavailability-enhancing agent, namely a solubilizing agent that helps to solubilize the free-B-ring flavonoid/flavan mixture (and any substantially water-insoluble oral care agents). In certain embodiments, the remainder of the humectant is preferably glycerine and/or sorbitol and/or xylitol. Water is typically present in amount of at least about 3% by weight; and glycerine and/or sorbitol and/or xylitol typically total about 6.5-75% by weight of the oral preparation, more typically about 10-75%, and, together with the solubilizing humectant, the humectant components typically amount to about 7-80% by weight of the oral preparation. Further, certain ingredients, are commercially provided in aqueous solutions (for example, sorbitol is a 70% aqueous solution). Where the composition contains a substantially water insoluble non-ionic antibacterial oral care agent, the composition has a minimal amount of polyethylene glycol, particularly of average molecular weight of 600 or more, since polyethylene glycol is believed to inhibit the antibacterial activity of certain noncationic antibacterial agent, even when another component, such as, propylene glycol is present to effect its solubilization.

In embodiments where an oral composition is in the form of a medicament, such as a non-abrasive gel or ointment, it can be applied to the gingival sulcus or margin and can be used in conjunction with wound dressings, gauze, films, and the like. Such gels may include both aqueous and non-aqueous gels. Aqueous gels generally comprise a polymer base, a thickener, a humectant, a flavoring agent, a sweetening agent, and a solvent, typically including water.

The present invention provides for methods and processes of using the oral compositions of the present invention to treat and inhibit oral conditions, such as oral inflammatory conditions, dental plaque, and dental calculus, all of which can lead to gingivitis and/or periodontitis. Further, the present invention provides for commercial packaging to distribute and store the oral compositions.

The oral compositions can be applied to the subject in any suitable manner, as is known in the art. For example, the oral compositions can be applied to the subject's oral cavity using a suitable applicator or delivery device, such as a brush, dental strip, film, syringe, tape, pill, or any other applicator or delivery device that is known in the art. The compositions can be used in prophylactic methods and processes to promote and maintain oral health, appearance, and breath freshness. The oral compositions can be repeatedly applied to the subject over a number of days according to a particular treatment schedule to treat and/or inhibit oral inflammatory conditions, dental plaque, or dental calculus. Instructions setting forth the treatment schedule can be provided in the commercial packaging.

The present invention is further illustrated through the following non-limiting example(s).

### Example 1

Dentifrice compositions of the present invention are made by combining the following ingredients as detailed in Tables 1 and 2:

**Table 1**

| **INGREDIENTS** | **DENTIFRICE 1** | **DENTIFRICE 2** |
|---|---|---|
| Sorbitol (70% Aqueous Solution) | 20.85 | 20.85 |
| Silica Abrasive | 20 | 20 |
| Glycerin | 20 | 20 |
| Water | 15.41 | 15.11 |
| GANTREZ® | 15 | 15 |
| Sodium Lauryl Sulfate | 1.5 | 1.5 |
| Amorphous Silica | 1.5 | 1.5 |
| Sodium Hydroxide | 1.2 | 1.2 |
| Sodium CMC | 1.1 | 1.1 |
| Flavor | 1 | 1 |
| Propylene Glycol | 0.5 | 0.5 |
| Titanium Dioxide | 0.5 | 0.5 |
| Carrageenan | 0.4 | 0.4 |
| Sodium Saccharin | 0.3 | 0.3 |
| Sodium Fluoride | 0.24 | 0.24 |
| triclosan | 0 | 0.3 |
| UNIVESTIN® | 0.5 | 0.5 |

**Table 2**

| | **DENTIFRICE** | **DENTIFRICE** | **DENTIFRICE** | **DENTIFRICE** | **DENTIFRICE** | **DENTIFRICE** |
|---|---|---|---|---|---|---|
| **INGREDIENTS** | **3** | **4** | **5** | **6** | **7** | **8** |
| Propylene Glycol | 2.0 | 2.0 | 2.0 | 2.0 | 2.0 | 2.0 |
| UNIVESTIN | 0.5 | 0.5 | 0.5 | --- | 0.5 | 0.5 |
| Anhydrous Citric Acid | --- | 0.6 | --- | --- | --- | --- |
| Trisodium Citrate Dihydrate | --- | 3.0 | --- | --- | --- | --- |
| Sodium Fluoride | 0.243 | --- | 0.243 | 0.243 | 0.243 | --- |
| Sodium Monoflurophosphate | --- | --- | --- | --- | --- | 0.243 |
| Stannous Fluoride | --- | 0.454 | --- | --- | --- | --- |
| Stannous Chloride | --- | 1.5 | 0.2 | 0.2 | 0.2 | 0.2 |
| Glycerin | 20.0 | 21.65 | 20.0 | 20.0 | 20.0 | 20.0 |
| Sodium CMC | 1.1 | 1.1 | 1.1 | 1.1 | 1.1 | 1.1 |
| Iota Carrageenan | 0.4 | --- | 0.4 | 0.4 | 0.4 | 0.4 |
| Sorbitol | 20.85 | 20.85 | 20.85 | 20.85 | 20.85 | 20.85 |
| Sodium Saccharin | 0.3 | 0.5 | 0.3 | 0.3 | 0.3 | 0.3 |
| Tetrasodium Pyrophosphate | --- | 2 | --- | --- | --- | --- |
| Titanium Dioxide | --- | 0.5 | --- | 0.5 | 0.5 | 0.5 |
| Sodium Hydroxide | 1.2 | q.s.* | 1.2 | 1.2 | 1.2 | 1.2 |
| GANTREZ® | --- | --- | --- | 2.0 | 2.0 | 2.0 |
| ZEODENT® 115-Abrasive | 10 | 10 | 10 | 20 | 20 | 20 |
| SYLODENT® XWA650-High Cleaning Silica | 10 | 10 | 10 | --- | --- | --- |
| ZEODENT® 165-Thickener | 1.5 | 3.5 | 1.5 | 1.5 | 1.5 | 1.5 |
| Flavor | 1.2 | 1.2 | 1.2 | 1.2 | 1.2 | 1.2 |
| triclosan | --- | --- | --- | 0.3 | 0.3 | 0.3 |
| Sodium Lauryl Sulfate | 1.5 | 1.5 | 1.5 | 1.5 | 1.5 | 1.5 |
| Water | Balance | Balance | Balance | Balance | Balance | Balance |

| | | | | | | |
|---|---|---|---|---|---|---|
| *Quantity sufficient to establish a pH of between about 5 - 8. | | | | | | |

The resulting dentifrice can be applied with a brush or other applicator to the oral surfaces.

The examples and other embodiments described herein are exemplary and not intended to be limiting in describing the full scope of compositions and methods of this invention. Equivalent changes, modifications and variations of specific embodiments, materials, compositions and methods may be made within the scope of the present invention, with substantially similar results.

## Claims

1. An oral composition comprising:
a free-B-ring flavonoid;
a flavan; and
an oral care active agent.

2. The composition according to claim 1, wherein the free-B-ring flavonoid and the flavan are present respectively in an amount of about 0.001% to about 5% by weight of the composition.

3. The composition according to claim 1, wherein the oral composition comprises the oral care active compound present in an amount of about 0.001 to about 5% by weight.

4. The composition according to claim 1, wherein the oral care active agent is selected from the group consisting of: an antibacterial agent, an antimicrobial agent, an anti-caries agent, an anti-tartar agent, an anti-attachment agent, a biofilm disruption agent, an anti-inflammatory agent, an antibiotic, and mixtures thereof.

5. The composition according to claim 1, wherein the oral care active agent comprises a non-ionic compound.

6. The composition according to claim 5, wherein the oral care active agent comprises 2',4,4'-trichloro-2-hydroxy-diphenyl ether (triclosan).

7. The composition according to claim 1, wherein the oral care active agent comprises a cationic active ingredient selected from the group consisting of:
benzethonium chloride, octenidine, hexetidine, hexamidine, cetyl pyridinium chloride, chlorhexidine, alexidine, N^{α}-cocoyl-L-arginine methyl ester, N^{α}-cocoyl-L-arginine ethyl ester, N^{α}-cocoyl-L-arginine propyl ester, N^{α}-stearoyl-L-arginine methyl ester, N^{α}-stearoyl-L-arginine ethyl ester, N^{α}-lauroyl arginine ethyl ester, 2',4,4'-trichloro-2-hydroxy-diphenyl ether (triclosan), stannous ion source, fluoride ion source, zinc ion source, salts and mixtures thereof.

8. The composition according to claim 1, further comprising a bioavailability-enhancing agent for the flavonoid and/or the flavan.

9. The composition according to claim 8, wherein the bioavailability-enhancing agent is at least one of a solubilizing agent and an efficacy-enhancing agent.

10. The composition according to claim 8, wherein the bioavailability-enhancing agent is one or more of: the efficacy-enhancing agent that is an anionic polymer or copolymer comprising a delivery-enhancing group and a retention-enhancing group, wherein the delivery-enhancing group enhances delivery of the flavonoid and the flavan to oral tissue and the retention-enhancing group enhances retention of the flavonoid and the flavan by oral tissue; and the solubilizing agent that is selected from an ether, a ketone, a glycol, a fat, an oil, a lipid, a surfactant, an alcohol, a humectant, a pyrrolidone, a polypyrrolidone, and mixtures thereof.

11. The composition according to claim 8, wherein the bioavailability-enhancing agent is selected from a polymeric polycarboxylate having an average molecular weight of about 100 to about 5,000,000 and an anionic polymeric phosphonate polymer having an average molecular weight of about 100 to about 1,000,000.

12. An oral composition according to claim 1, wherein the oral care active agent comprises a stannous ion source.

13. The composition according to claim 12, wherein the stannous ions are present in an amount of about 0.1 to about 10% by weight of the composition.

14. The composition according to claim 12, wherein the stannous ion source is selected from the group consisting of: stannous fluoride, stannous chloride, stannous pyrophosphate, stannous formate, stannous acetate, stannous gluconate, stannous lactate, stannous tartrate, stannous oxalate, stannous malonate, stannous citrate, stannous ethylene glyoxide, and mixtures thereof.

15. The composition according to claim 12, further comprising one or more of a fluoride ion source and a zinc ion source.

16. The composition according to claim 12, further comprising an additional oral care active agent.

17. The composition according to claim 16, wherein the additional oral care active agent is selected from the group consisting of: an antibacterial agent, an antimicrobial agent, an anti-caries agent, an anti-tartar agent, an anti-attachment agent, a biofilm disruption agent, an anti-inflammatory agent, an antibiotic, and mixtures thereof.

18. The composition according to claim 12, further comprising a bioavailability-enhancing agent for the flavonoid and/or the flavan comprising at least one of: an efficacy-enhancing agent and a solubilizing agent.

19. The composition according to claim 12 further comprising a carrier including a colorant, wherein the stannous ion source promotes color stability of the oral composition.

20. An oral composition according to claim 1, wherein the oral care active agent comprises a cationic compound.

21. The composition according to claim 20, wherein the cationic compound is selected from the group consisting of: benzethonium chloride, octenidine, hexetidine, hexamidine, cetyl pyridinium chloride, chlorhexidine, alexidine, N^{α}-cocoyl-L-arginine methyl ester, N^{α}-cocoyl-L-arginine ethyl ester, N^{α}-cocoyl-L-arginine propyl ester, N^{α}-stearoyl-L-arginine methyl ester, N^{α}-stearoyl-L-arginine ethyl ester, N^{α}-lauroyl arginine ethyl ester, salts and mixtures thereof.

22. The composition according to claim 20, wherein the oral care active agent comprises ethyl lauroyl arginine hydrochloride (ELAH).

23. The composition according to claim 20, further comprising a bioavailability-enhancing agent for the flavonoid and/or the flavan comprising at least one of: an efficacy-enhancing agent and a solubilizing agent.

24. An oral composition for use in a method for inhibiting and/or treating an oral inflammatory condition in a mammalian subject, the oral composition comprising a free-B-ring flavonoid, a flavan, and a bioavailability-enhancing agent for increasing the bioavailability of the flavonoid and/or the flavan in the oral tissue in the oral cavity, wherein the method comprises administering to the oral cavity of the subject an effective amount of the oral composition.

25. The oral composition according to claim 24, wherein the free-B-ring flavonoid and the flavan are present respectively in an amount of about 0.001% to about 5% by weight of the composition.

26. The oral composition according to claim 24, wherein the bioavailability-enhancing agent is selected from a polymeric polycarboxylate having an average molecular weight of about 100 to about 5,000,000 and an anionic polymeric phosphonate polymer having an average molecular weight of about 100 to about 1,000,000.

27. The oral composition according to claim 24, wherein the composition further comprises an oral care active agent selected from the group consisting of: an antibacterial agent, an antimicrobial agent, an anti-caries agent, an anti-tartar agent, an anti-attachment agent, a biofilm disruption agent, an anti-inflammatory agent, an antibiotic, and mixtures thereof.
